# EUROPÄISCHE PATENTSCHRIFT

(11) **EP 0 340 159 B1**
(45) Veröffentlichungstag und Bekanntmachung des Hinweises auf die Patenterteilung: **20.01.1993**
(21) Anmeldenummer: 89810231.4
(22) Anmeldetag: 23.03.1989
(51) Int. Cl.: A61B 17/58

(54) **Spreizdübel für eine zementfreie Verankerung von Knochenimplantaten**
Dowel pin for cementless bone implants
Cheville à expansion pour l'ancrage sans ciment d'une prothèse osseuse

(30) Priorität: 27.04.1988 CH 1578/88
(43) Veröffentlichungstag der Anmeldung: 02.11.1989
(73) Patentinhaber: GEBRÜDER SULZER AKTIENGESELLSCHAFT, CH-8401 Winterthur (CH); ALLO PRO AG, 6340 Baar (CH)
(72) Erfinder: Karpf, Kurt, CH-4718 Holderbank (CH)

(56) Entgegenhaltungen:
- DE-U- 8 520 206
- US-A- 4 013 071
- US-A- 4 611 581

## Beschreibung

Die Erfindung betrifft einen Spreizdübel für eine zementfreie Verankerung von Knochenimplantaten, bestehend aus einem einseitig offenen, mit Hilfe von Längsschlitzen aufspreizbaren Hohlkörper und einem in den Hohlkörper eintreibbaren Spreizkörper, wobei der Hohlkörper auf seiner Aussenmantelfläche widerhakenartige, zirkuläre Verzahnungen trägt.

Derartige Spreizdübel sind beispielsweise bekannt aus der CH-B 662 501. Bei dieser Konstruktion besteht der Spreizkörper aus einem in Richtung der Längsachse einschlagbaren Zapfen, bei dessen Eintreiben keine Drehmomente auf den Dübel ausgeübt werden. Sehr häufig sollen mit Implantat-Spreizdübeln jedoch, wie in anderen Gebieten der Technik, Schrauben verankert werden. Bei den bisherigen Konstruktionen der Dübel ist beim Einschrauben der Schraube häufig die Schwierigkeit aufgetreten, dass der von der Schraube noch nicht oder nur wenig aufgespreizte Dübel sich in der Knochenbohrung "mitdreht", und sich somit keine feste Verankerung des Dübels ergibt. Als Folge davon wird selbstverständlich das Implantat ungenügend fixiert, das beispielsweise aus Kontraktions-oder Distraktionsplatten besteht, die zur Behandlung von Wirbelsäulen-Deformationen zwischen zwei Wirbeln ausgespannt und in diesen durch Spreizdübel mit Schrauben gehalten werden.

Aufgabe der Erfindung ist es daher, einen Spreizdübel zu schaffen, der unbeabsichtigten Rotationen, beispielsweise beim Einschrauben einer Schraube, widersteht. Diese Aufgabe wird dadurch gelöst, dass der äussere Umfang des Hohlkörpers mindestens über eine Teillänge des Dübels im Querschnitt polygonförmig ausgebildet ist.

Bei der Implantation wird die Bohrung für die Aufnahme des neuen Dübels auf einen Durchmesser ausgebohrt, der dem Abstand zweier einander gegenüberliegender Seiten entspricht, so dass die Kanten des Polygonzuges bereits in die Knochensubstanz eindringen. Die Polygonform der Verzahnungen des in die Bohrung eingesetzten Hohlkörpers des Dübels wirkt dann als Drehsicherung gegen unbeabsichtigte Rotationen des Dübels.

Bei sehr vielen Knochen variieren Knochendichte und Knochenhärte sehr stark, wie beispielsweise beim Wirbel, bei dem der Wirbelbogen aus harter, dichter, kortikaler Knochensubstanz und der Wirbelkörper in seinem Inneren aus relativ weichem und "luftigem" spongiösen Material bestehen. In an sich bekannter Weise kann bei dem neuen Dübel diesen Materialunterschieden dadurch Rechnung getragen werden, dass der Hohlkörper nahe dem geschlossenen Ende der Längsschlitze eine Feinverzahnung trägt, an die zum offenen Ende hin eine Grobverzahnung anschliesst.

Beim Eindringen der Grobverzahnung in das relativ weiche spongiöse Material besteht die Gefahr, dass die Zahnspitzen unter Biegebelastungen allmählich immer weiter in den Knochen eindringen; das hat zur Folge, dass die in den Dübel eingeschraubte Schraube immer weiter in den Dübel "versinkt" und schliesslich locker wird. In diesem Fall bewirkt die Polygonform zusätzlich, dass die Biegebelastungen über eine relativ breite Fläche auf den Knochen übertragen werden; darüberhinaus lässt sich diese Wirkung der Polygonform in dem relativ weichen spongiösen Knochengewebe verstärken, wenn die Zähne der Grobverzahnung zu achsparallelen Flächen abgestumpft sind.

Weiterhin kann die Verankerung des Dübels im spongiosen Material verbessert werden, wenn der axiale Abstand der Zähne der Grobverzahnung grösser ist als ihre Zahntiefe.

Um die Anpressung des Dübels in unterschiedlichen Knochensubstanzen zu vergleichmässigen, ist es weiterhin zweckmässig, wenn die Innenbohrung des Hohlkörpers im Bereich der Feinverzahnung zylindrisch und im Bereich der Grobverzahnung konisch zulaufend ausgebildet ist.

Im folgenden wird die Erfindung anhand von Ausführungsbeispielen im Zusammenhang mit der Zeichnung näher erläutert.
Fig. 1 ist eine Seitenansicht einer Ausführungsform des neuen Dübels;
Fig. 2 ist eine Aufsicht auf Fig. 1 von oben;
Fig. 3 gibt einen Längsschnitt durch eine zweite Ausführungsform des neuen Dübels wieder;
Fig. 4 ist eine zu dem Dübel nach Fig. 3 passende Aufspreizschraube;
Fig. 5 schliesslich stellt schematisch den neuen Dübel, eingesetzt in einen Wirbel dar.

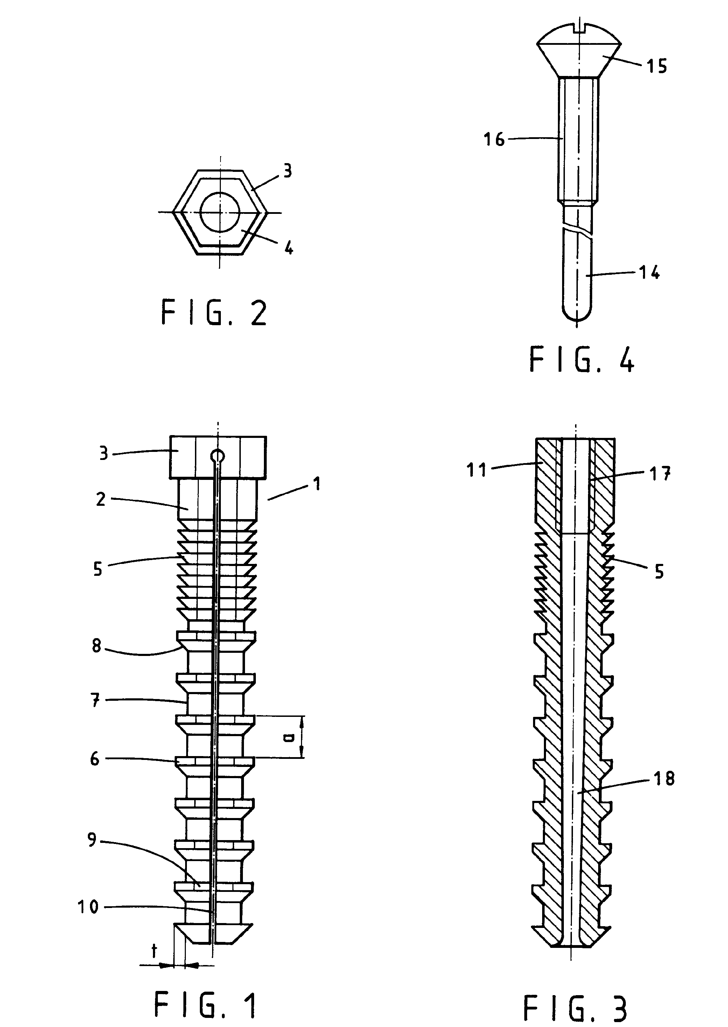

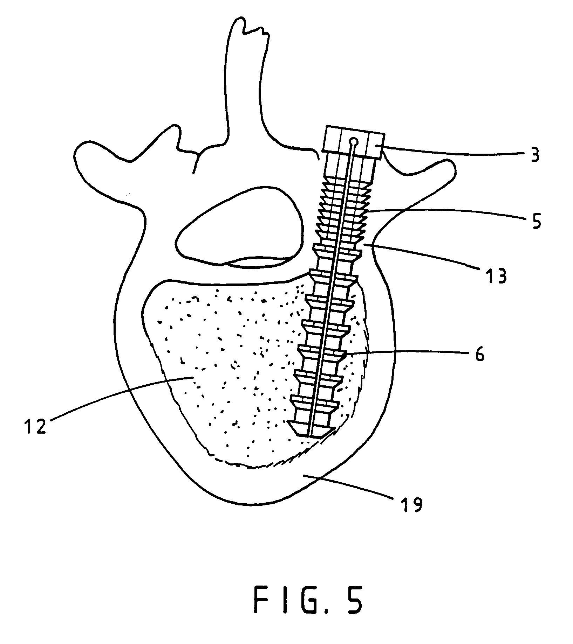

Der beispielsweise aus körperverträglichem Kunststoff, üblicherweise aus Polyäthylen oder aus einem Metall, bestehende Hohlkörper 1 des Spreizdübels nach Fig. 1 hat einen im Durchmesser gegenüber dem Kern 2 vergrösserten Kopf 3, der neben der in diesem Fall sechskantigen Aussenform einen Innensechskant 4 zur Aufnahme eines entsprechenden, nicht gezeigten Setz- oder Einschlaginstrumentes aufweist. Nach unten setzt sich der ebenfalls sechskantige Kern 2 in einer Feinverzahnung 5 fort. An die Feinverzahnung 5 schliesst eine Grobverzahnung 6 an, die sich bis zum freien Ende des Dübels erstreckt. Sowohl die Feinverzahnung 5 als auch die Grobverzahnung 6 sind in Umfangsrichtung ebenfalls polygonförmig, im vorliegenden Fall wiederum als Sechskant, ausgebildet. In dem in Fig. 1 gezeigten Beispiel ist nur der obere Bereich des Kernes 2, der im Durchmesser den Durchmessern der Verzahnungen 5 und 6 entspricht, polygonförmig gestaltet, während der Stamm 7, aus dem die Grobverzahnung 6 "herauswächst", eine zylindrische Form hat. Selbverständlich ist es jedoch auch möglich, den Stamm 7 und die Flanken 8 der Grobverzahnung 6 als Polygone auszubilden. Der Abstand a der Zähne der Grobverzahnung 6 ist grösser als die Zahntiefe t, damit zwischen den einzelnen Zahnreihen möglichst viel Knochensubstanz der relativ weichen Spongiosa 12 (Fig. 5) für die Verankerung zur Verfügung steht.

Die Zähne der Grobverzahnung 6 sind an ihren Spitzen zu ebenen achsparallen Flächen 9 abgestumpft; diese Flächen bewirken eine Verteilung der von Biegebelastung herrührenden Drücke oder Kräfte auf einen relativ grossen Bereich des weichen Knochengewebes 12, in das die Grobverzahnung 6 zu liegen kommt.

Die Spreizung des Hohlkörpers 1 wird durch Längsschlitze 10 erreicht, die sich vom freien Ende bis in den Kopf 3 erstrecken und von denen im allgemeinen mindestens zwei auf dem Umfang verteilt sind.

Obwohl das Aufspreizen des Hohlkörpers 1 auch durch Einschlagen eines Zapfens erfolgen kann, werden dafür Spannschrauben bevorzugt, die nahe ihrem Kopf 15 (Fig. 4) über eine gewisse Länge ein Gewinde 16 tragen, an das sich ein gewindefreier Stamm 14 anschliesst. Das Gewinde 16 wird zum Aufspreizen des Hohlkörpers 11, der als Unterschied zum Hohlkörper 1 von Fig. 1, lediglich keinen Kopf mit vergrössertem Durchmesser aufweist, in ein Gewinde 17 der Längsbohrung 18 des Hohlkörpers 11 eingeschraubt. Im Bereich des Gewindes 17 und im anschliessenden Teil, der aussen die widerhakenartige Feinverzahnung 5 trägt, ist die Längsbohrung 18 zylindrisch, während sie sich im Bereich der Grobverzahnung 6 zum freien Ende hin konisch verjüngt.

Die in Fig. 5 dargestellte Anwendung des neuen Spreizdübels zeigt, dass sich die Feinverzahnung 5 vor allem im harten kortikalen Gewebe 13 des Wirbelbogens erstreckt, während die Grobverzahnung 6 in das weiche spongiose Gewebe 12 im Innern des Wirbelkörpers 19 verläuft.

Selbstverständlich ist die Polygonform des Querschnittes nicht auf diejenige eines Sechskantes beschränkt; sie kann in gleicher Weise auch eine beliebige andere Kantenzahl haben.

## Patentansprüche

1. Spreizdübel für eine zementfreie Verankerung von Knochenimplantaten, bestehend aus einem einseitig offenen, mit Hilfe von Längsschlitzen (10) aufspreizbaren Hohlkörper (1,11) und einem in den Hohlkörper eintreibbaren Spreizkörper, wobei der Hohlkörper auf seiner Aussenmantelfläche widerhakenartige, zirkuläre Verzahnungen (5,6) trägt, **dadurch gekennzeichnet, dass** der äussere Umfang des Hohlkörpers (1, 11) im Bereich der Verzahnungen mindestens über eine Teillänge des Dübels im Querschnitt polygonförmig ausgebildet ist.

2. Spreizdübel nach Anspruch 1, **dadurch gekennzeichnet, dass** der Hohlkörper (1,11) nahe dem geschlossenen Ende der Längsschlitze (10) eine Feinverzahnung (5) trägt, an die zum offenen Ende hin eine Grobverzahnung (6) anschliesst.

3. Spreizdübel nach Anspruch 2, **dadurch gekennzeichnet, dass** die Zähne der Grobverzahnung (6) zu achsparallelen Flächen (9) abgestumpft sind.

4. Spreizdübel nach Anspruch 2 oder 3, **dadurch gekennzeichnet, dass** der axiale Abstand (a) der Zähne der Grobverzahnung (6) grösser ist als ihre Zahntiefe (t).

5. Spreizdübel nach einem der Ansprüche 2 bis 4, **dadurch gekennzeichnet, dass** die Innenbohrung (18) des Hohlkörpers (11) im Bereich der Feinverzahnung (5) zylindrisch und im Bereich der Grobverzahnung (6) konisch zulaufend ausgebildet ist.

## Claims

1. An expanding dowel for cementless anchoring of bone implants, comprising a hollow member (1, 11) which is open on one side and can be expanded by means of longitudinal slots (10), and an expanding member which can be driven into the hollow member, the hollow member bearing sets of barb-like circular teeth (5, 6) on its outer generated surface, characterised in that the outer periphery of the hollow member (1, 11) is polygonal in cross-section in the vicinity of the teeth at least over part of the length of the dowel.

2. An expanding dowel as claimed in claim 1, characterised in that the hollow member (1, 11) bears near the closed end of the longitudinal slots (10) a set of fine teeth (5) which is adjoined on the side nearer the open end by a set of coarse teeth (6).

3. An expanding dowel as claimed in claim 2, characterised in that the teeth in the coarse set (6) are blunted to make surfaces (9) parallel to the axis

4. An expanding dowel as claimed in claim 2 or 3, characterised in that the axial spacing a of the teeth in the coarse set (6) is greater than their tooth depth t.

5. An expanding dowel as claimed in any of claims 2 to 4, characterised in that the internal bore (18) in the hollow member (11) is cylindrical in the vicinity of the fine tooth set (5) and tapers conically in the vicinity of the coarse set (6).

## Revendications

1. Cheville à expansion pour la fixation sans ciment de prothèses dans des os, se composant d'un corps creux (1, 11) ouvert d'un côté, expansible grâce à des fentes longitudinales (10), et d'un corps expanseur destiné à être chassé dans le corps creux, le corps creux comportant à la surface de l'enveloppe extérieure des dentures circulaires (5, 6) en forme de crochets de retenue, caractérisée en ce que la circonférence extérieure du corps creux (1, 11) a une section polygonale dans la région des dentures, au moins sur une partie de la longueur de la cheville.

2. Cheville à expansion selon la revendication 1, caractérisée en ce que le corps creux (1, 11) comporte, à proximité de l'extrémité fermée des fentes longitudinales (10), une denture fine (5) à laquelle fait suite vers l'extrémité ouverte une denture (6) à grands entre-dents.

3. Cheville à expansion selon la revendication 2, caractérisée en ce que les dents de la denture (6) à grands entre-dents sont tronquées de manière à former des surfaces (9) parallèles à l'axe.

4. Cheville à expansion selon la revendication 2 ou 3, caractérisée en ce que la distance axiale (a) séparant les dents de la denture (6) à grands entre-dents est supérieure à la profondeur (t) des dents.

5. Cheville à expansion selon l'une des revendications 2 à 4, caractérisée en ce que le trou intérieur (18) du corps creux (11) est cylindrique dans la région de la denture fine (5) et va en se rétrécissant en cône dans la région de la denture (6) à grands entre-dents.
